**Europäisches Patentamt**

**European Patent Office**

**Office européen des brevets**

(19)

(11) Veröffentlichungsnummer: **0 002 706**
**B1**

(12) **EUROPÄISCHE PATENTSCHRIFT**

(45) Veröffentlichungstag der Patentschrift: **29.04.81**

(51) Int. Cl.³: **A 61 B 6/14, A 61 B 6/04**

(21) Anmeldenummer: **78101617.5**

(22) Anmeldetag: **08.12.78**

(54) Zahnärztliche Röntgendiagnostikeinrichtung.

(30) Priorität: **27.12.77 DE 2758191**

(43) Veröffentlichungstag der Anmeldung:
**11.07.79 Patentblatt 79/14**

(45) Bekanntmachung des Hinweises auf die Patenterteilung:
**29.04.81 Patentblatt 81/17**

(84) Benannte Vertragsstaaten:
**DE FR GB IT**

(56) Entgegenhaltungen:
**DE - A - 2 335 918**
**DE - A - 2 343 862**
**DE - A - 2 404 469**
**DE - A - 2 461 441**
**FR - A - 2 189 001**
**NL - A - 78 06578**
**NL - A - 78 06579**
**US - A - 3 906 227**
**US - A - 3 916 192**
**US - A - 3 974 388**
**US - A - 4 019 059**

(73) Patentinhaber: **SIEMENS AKTIENGESELLSCHAFT**
**Berlin und München**
**Postfach 22 02 61**
**D-8000 München 22 (DE)**

(72) Erfinder: **Neuendorf, Manfred**
**Ginsterweg 3**
**D-6143 Lorsch (DE)**
Erfinder: **Schmitt, Ernst**
**Altengassweg 42**
**D-6140 Bensheim (DE)**
Erfinder: **Schubert, Gustav**
**Hügelstrasse 55**
**D-6143 Lorsch (DE)**

Courier Press, Leamington Spa, England.

## Zahnärztliche Röntgendiagnostikeinrichtung

Die Erfindung bezieht sich auf eine zahnärztliche Röntgendiagnostikeinrichtung mit einer schwenkbar gehaltenen Tragvorrichtung für eine Röntgenröhre und einen Filmträger und mit einem dazwischen angeordneten, zur Auf- oder Anlage eines Patientkopfes dienenden Kopfhalter, der, um den Kopf für verschiedene Objektaufnahmen in unterschiedlichen Positionen fixieren zu können, in horizontaler Ebene gegenüber einer Halterung verstellbar angeordnet ist.

Aus der DE—A—23 35 918 ist eine zahnärztliche Röntgendiagnostikeinrichtung mit einem Kopfhalter bekannt, der zwei in die beiden Ohren des Patienten einsetzbare Ohrbolzen enthält. Mittels einer Verstelleinrichtung können die Ohrbolzen ohne Änderung ihrer gegenseitigen Lage mit Bezug auf die Projektionsrichtung des Röntgenstrahls der Röntgenröhre verstellt werden.

Um unterschiedlichen Aufnahmetechniken im Kieferbereich eines Patienten gerecht zu werden, z.B. um Aufnahmen des Zahnbogens, der Sinus-Höhlen, der Kiefergelenke oder Gehörgänge machen zu können, ist es notwendig, den Kopfhalter, der auch mit einer Kinnstütze, einem Aufbißblock oder mit einer Frontzahnanlage bestückt sein kann, zumindest in horizontaler Ebene in verschiedene Positionen einstellen zu können.

Bei einer im Handel unter der Bezeichnung "ORTHOPANTHOMOGRAPH" entsprechend dem SIEMENS-Prospektblatt WS 4778 (MD 80/1155) bekannten Röntgendiagnostikeinrichtung ist an einem in einer Führungsschiene verschiebbar gehaltenen Kopfhalter ein Zeiger befestigt, der mit einer an der Halterung angeordneten Strichskala zusammenwirkt. Die jeweils eingestellte Position des Kopfhalters kann an der Strichskala abgelesen werden.

Bei der Einstellung der verschiedenen Positionen kann es leicht vorkommen, daß der Zeiger versehentlich auf einen falschen Skalenwert und damit der Kopfhalter nicht in der für die gewünschte Aufnahme zugehörigen Position eingestellt wird. Das Aufsuchen des für eine gewünschte Aufnahme jeweils zugehörigen Skalenwertes ist außerdem relativ zeitaufwendig.

Ziel der Erfindung ist es, eine demgegenüber verbesserte Röntgendiagnostikeinrichtung anzugeben, die ein leichteres und sichereres Einstellen der für die jeweiligen Aufnahmetechniken erforderlichen Positionen des Kopfhalters ermöglicht.

Das angestrebte Ziel wird gemäß der Erfindung bei einer zahnärztlichen Röntgendiagnostikeinrichtung der eingangs genannten Art dadurch erreicht, daß die einstellbaren Positionen des Kopfhalters entsprechend den verschiedenen Objektaufnahmen auf einem Übersichtsfeld markiert sind daß jede Position durch einen im Übersichtsfeld angeordneten Positionswähler anwählbar ist, und daß mit den Positionswählern eine Abtastvorrichtung mit Signalgebern gekuppelt ist, durch die die Lage des Kopfhalters in bezug auf seine Halterung erfaßt wird und die bei Erreichen einer angewählten Position ein Signal an ein im Übersichtsfeld angeordnetes und dem betreffenden Positionswähler zugeordnetes Anzeigeglied gibt.

Vorteilhafte Ausgestaltungen und Weiterbildungen sind in den Unteransprüchen enthalten.

Ein Ausführungsbeispiel der Erfindung wird nachstehend anhand der Zeichnung näher erläutert.
Es zeigen:
Fig. 1 die wesentlichen Teile einer zahnärztlichen Röntgendiagnostikeinrichtung in schaubildlicher Ansicht,
Fig. 2 und 3 Einzelheiten der in Fig. 1 gezeigten Einrichtung,
Fig. 4 ein Prinzipschaltbild.

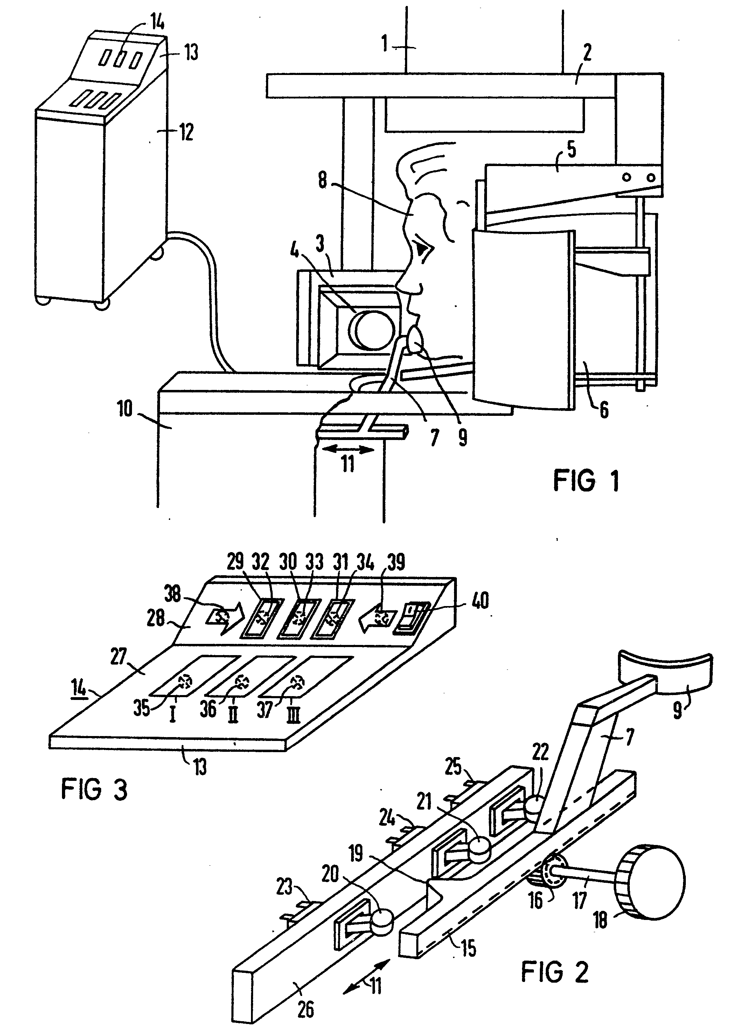

Die Fig. 1 zeigt in einer schaubildlichen Übersichtsdarstellung die wesentlichen Teile einer zahnärztlichen Röntgendiagnostikeinrichtung, wie sie für Übersichtsaufnahmen im Zahn-Kieferbereich verwendet wird. Die Einrichtung besteht im wesentlichen aus einer an einer Tragsäule 1 schwenkbar gehalterten Tragvorrichtung 2 für einen ersten Tragarm 3, an dem eine Röntgenröhre 4 gehaltert ist, und einem weiteren Tragarm 5, an dem ein bogenförmig gekrümmter Filmträger 6 gehaltert ist. Zwischen dem Filmträger 6 und der Röntgenröhre 4 befindet sich ein Kopfhalter 7 zur Auf- oder Anlage eines Patientenkopfes 8. Der Kopfhalter 7 ist im vorliegenden Ausführungsbeispiel mit einer Kinnstützte 9 bestückt; anstelle der Kinnstütze kann auch ein Aufbißblock, der in den Patientenmund eingeführt wird, oder auch eine Frontzahnanlage vorgesehen sein.

Zur Anfertigung einer Zahn- oder Kieferaufnahme wird der Kopf 8 des Patienten am Kopfhalter 7 fixiert. Der besseren Übersichtlichkeit wegen sind weitere, zur Fixierung und Justierung des Kopfes evtl. notwendige Vorrichtungen hier nicht eingezeichnet. Während sich nach einem nicht näher erläuterten Drehsystem die Röntgenröhre 4 und der Filmträger 6 um den Patientenkopf 8 herumbewegen, muß dieser während der Aufnahme an der Kopfhalterung fest an- bzw. aufliegen.

Um nun verschiedenen Aufnahmetechniken gerecht zu werden, z.B. um sowohl Zahnbogen-, Sinus-Höhlen-, Kiefergelenk- oder Gehörgang-Aufnahmen machen zu können, muß der Patientenkopf 8 in verschiedene Positionen in der Verlängerung der Schichtbogenachse (darunter wird eine zwischen der Frontzähnen hindurch und in Richtung Halswirbelsäule verlaufende gedachte Linie verstanden) gebracht werden können. Hierzu ist der Kopfhalter 7 in einem mit 10 bezeichneten Gehäuse in Richtung des Pfeiles 11 verstellbar gehalten.

Mit 12 ist eine fahrbare Einheit bezeichnet, die die zum Betrieb der Röntgendiagnostikeinrichtung erforderlichen elektrischen Anlagen enthält. Deckseitig der Einheit 12 ist ein pultförmig ausgebildetes Teil 13 aufgesetzt, welches ein mit verschiedenen Markierungs und Anzeigegliedern bestücktes Übersichtsfeld 14 aufweist.

Die Fig. 2 zeigt Einzelheiten über den Aufbau und die Lagerung des Kopfhalters 7. Der Kopfhalter 7 weist eine Zahnstange 15 auf, die mit einem Zahnrad 16 kämmt, welches über eine Achse 17 mit einem Antrieb 18 (Handrad oder motorischer Antrieb) angetrieben wird. Die Zahnstange 15 weist eine Schaltnocke 19 auf, die bei Verstellung der Zahnstange in Richtung des Pfeiles 11 Hebel 20 bis 22 betätigt, die mit Mikroschaltern 23 bis 25 zusammenwirken, welche auf einer Halterung 26 in einem bestimmten Abstand angeordnet sind, der der erforderlichen Patientenkopfpositionierung für die betreffende Aufnahme entspricht.

Die Fig. 3 zeigt Einzelheiten des pultförmigen Gehäuses 13. Das Gehäuse 13 weist einen im wesentlichen horizontal verlaufenden Abschnitt 27 und einen schräg dazu verlaufenden Abschnitt 28 auf. Im Abschnitt 27 sind drei einstellbare Patientenkopfpositionierungen I, II, III markiert. Die Markierung I kann z.B. eine Normalaufnahme, die Markierung II eine Sinushöhlenaufnahme und die Markierung III eine Kiefergelenkaufnahme symbolisieren. Den durch die Markierungen I bis III entsprechenden Aufnahmepositionen sind Positionswähler 29 bis 31 in Form von Schaltern zugeordnet, die jeweils durch Anzeigelampen 32 bis 34, wie später noch näher erläutert, optisch hervorgehoben werden. Die Patientenpositionierungsmarkierungen I bis III weisen ebenfalls Anziegelampen 35 bis 37 auf, die bei Erreichen der durch die Positionswähler 29 bis 31 vorgewählten Position aufleuchten. Mit 38 und 39 sind zwei Lichtpfeile bezeichnet, die die Bewegungsrichtung des Kopfhalters 7 während einer Verstellung anzeigen. Die elektrische Anlage wird mit dem Hauptschalter 40 eingeschaltet.

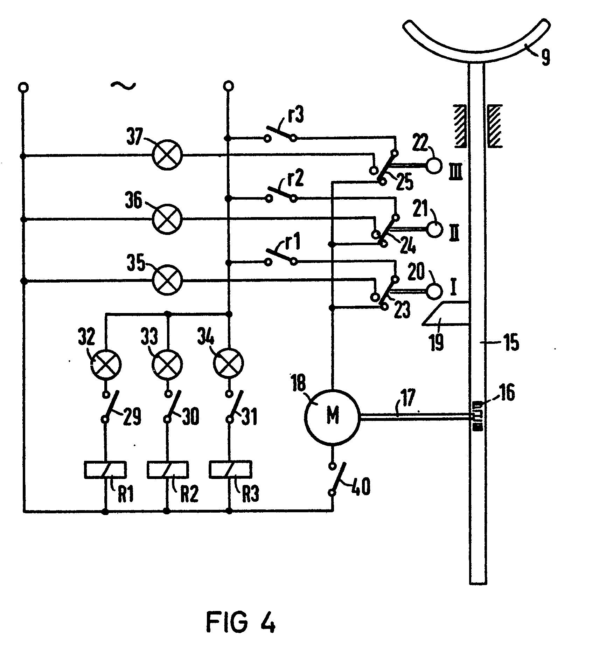

Aus der Fig. 4, die ein einfaches Prinzipschaltbild Anlage zeigt, geht hervor, daß nach Betätigen eines der Positionswähler 29 bis 31 die zugehörige Anzeigelampe aufleuchtet. Gleichzeitig wird das zugehörige Relais $R_1$, $R_2$ bzw. $R_3$ mit den Kontakten $r_1$, $r_2$ bzw. $r_3$ erregt. Vorausgesetzt, daß der Hauptschalter 40 eingeschaltet ist, wird der Antriebsmotor 18 eingeschaltet und sodann die Zahnstange 15 in Pfeilrichtung bewegt. Bei Erreichen der dem eingeschalteten Positionswähler zugehörigen Patientenposition leuchtet die entsprechende Patientenpositionsanzeigelampe 35, 36 oder 37 auf. Die Bedienungsperson erhält dadurch die Information, daß der Kopfhalter entsprechend der gewählten Aufnahmetechnik in der richtigen Position sich befindet. Gleichzeitig erfolgt über die Schalter 23, 24 bzw. 25 das Abschalten des Antriebs 18.

Anstelle des Nockens 19 in Verbindung mit den Mikroschaltern 23 bis 25 kann auch eine rein optische Abtastvorrichtung mittels Lampen und Fotowiderständen vorgesehen werden, die über einer Verstärker die Anzeigelampen in der beschriebenen Weise im Übersichtsfeld 14 ansteuern. Die Stromversorgung erfolgt für beide Varianten zweckmäßigerweise mit Niederspannung (Wechsel- oder Gleichspannung).

In dem Prinzipschaltbild nach Fig. 4 sind die für die Drehrichtungsumkehr des Antriebs erforderlichen Schaltungsteile, insbesondere die Selbsthaltungs- und Verriegelungsschaltungsteile, der besseren Übersichtlichkeit wegen nicht eingezeichnet.

## Patentansprüche

1. Zahnärztliche Röntgendiagnostikeinrichtung mit einer schwenkbar gehaltenen Tragvorrichtung für eine Röntgenröhre (4) und einer Filmträger (6) und mit einem dazwischen angeordneten, zur Auf- oder Anlage eines Patientenkopfes (8) dienenden Kopfhalter (7), der, um den Kopf für verschiedene Objektaufnahmen in unterschiedlichen Positionen fixieren zu können, in horizontaler Ebene gegenüber einer Halterung (10) verstellbar angeordnet ist, dadurch gekennzeichnet, daß die einstellbaren Positionen (I, II, III) des Kopfhalters (7) entsprechend den verschiedenen Objektaufnahmen auf einem Übersichtsfeld (14) markiert sind, daß jede Position durch einen im Übersichtsfeld (14) angeordneten Positionswähler (29 bis 31) anwählbar ist, und daß mit den Positionswählern (29, 30, 31) eine Abtastvorrichtung (19 bis 25) mit Signalgebern (23, 24, 25) gekuppelt ist, durch die die Lage des Kopfhalters (7) in bezug auf seine Halterung (26) erfaßt wird und die bei Erreichen einer angewälten Position (I bis III) ein Signal an ein im Übersichtsfeld (14) angeordnetes und dem betreffenden Positionswähler (29 bis 31) zugeordnetes Anzeigeglied (35 bis 37) gibt.

2. Röntgendiagnostikeinrichtung nach Anspruch 1, dadurch gekennzeichnet, daß eine mechanische Abtasteinrichtung (19 bis 25) mit elektrischen Schaltern (23 bis 25) als Signalgeber vorgesehen ist.

3. Röntgendiagnostikeinrichtung nach Anspruch 2, dadurch gekennzeichnet, daß die Abtasteinrichtung (19 bis 25) im wesentlichen aus einer an einer den Kopfhalter (7) tragenden Stange (15) angeordneten Schaltnocke (19) und an der Halterung (26) angeordneten und mit Mikroschaltern (23 bis 25) zusammenwirkenden Schalthebeln (20 bis 22) besteht.

4. Röntgendiagnostikeinrichtung nach Anspruch 3, dadurch gekennzeichnet, daß die Stange (15) als Zahnstange ausgebildet ist, die über ein von Hand oder elektromotorisch angetriebenes Zahnrad (16) verstellt wird.

5. Röntgendiagnostikeinrichtung nach Anspruch 1, dadurch gekennzeichnet, daß die Markierungen für die einstellbaren Positionen (I, II, III) optische Anzeigeglieder, vorzugsweise Anzeigeleuchten (35 bis 37) aufweisen.

6. Röntgendiagnostikeinrichtung nach Anspruch 5, dadurch gekennzeichnet, daß auch die Positionswähler (29 bis 31) optische Anzeigeglieder, vorzugsweise Anzeigeleuchten (32 bis 34), enthalten, die bei Betätigung der Positionswähler, (29 bis 31) ansprechen.

7. Röntgendiagnostikeinrichtung nach einem der Ansprüche 1 bis 6, dadurch gekennzeichnet, daß das Übersichtsfeld (14) an der Oberseite eines pultförmig ausgebildeten Gehäuses (13) angeordnet ist.

8. Röntgendiagnostikeinrichtung nach Anspruch 7, dadurch gekennzeichnet, daß das Gehäuse (13) einen waagerechten Teil (27) aufweist, in dem die Markierungen der einzelnen Positionen (I, II, III) angeordnet sind und die Positionswähler (29 bis 31) im schräg verlaufenden Teil (28) angeordnet sind.

9. Röntgendiagnostikeinrichtung nach einem der Ansprüche 1 bis 8, dadurch gekennzeichnet, daß im Übersichtsfeld (14) Lichtpfeile (38, 39) angeordnet sind, welche die Bewegungsrichtung des Kopfhalters (7) während der Verstellung anzeigen.

## Revendications

1. Appareil de radiodiagnostic dentaire comportant un dispositif de support monté de façon à pouvoir pivoter et prévu pour un tube à rayons X (4) et un support de film (6), et comportant un appui-tête (7) disposé entre le tube à rayons X et le support de film et servant au soutien ou à l'appui pour la tête (8) d'un patient et qui est disposé de façon à pouvoir être déplacé dans un plan horizontal par rapport à un support (10), afin de pouvoir bloquer la tête dans des positions différentes pour des radiographies d'objets différentes, caractérisé par le fait que les positions réglables (I, II, III) de l'appui-tête (7) sont marquées conformément aux différentes radiographies d'objets sur un panneau d'ensemble (14), que chaque position peut être sélectionnée au moyen d'un sélecteur de position (29 à 31) disposé dans le panneau d'ensemble (14), et qu'aux sélecteurs de position (29, 30, 31) est-accouplé un dispositif d'exploration (19 à 25) comportant des générateurs de signaux (23, 24, 25) et à l'aide duquel la position de l'appui-tête (7) est détectée par rapport à son support (26) et qui délivre un signal à un organe d'affichage (35 à 37) disposé dans le panneau d'ensemble (14) et associé au sélecteur de position concerné (29 à 31), lorsqu'une position sélectionnée (I à III) est atteinte.

2. Appareil de radiodiagnostic suivant la revendication 1, caractérisé par le fait qu'il est prévu comme générateur de signaux un dispositif mécanique d'exploration (19 à 25) comportant des commutateurs électriques (23 à 25).

3. Appareil de radiodiagnostic suivant la revendication 2, caractérisé par le fait que le dispositif d'exploration (19 à 25) est constitué essentiellement par une came de commutation (19) montée sur une barre (15) portant l'appui-tête (7), et par des leviers de commutation (20 à 22) disposés sur le support (26) et coagissant avec des microcommutateurs (23 à 25).

4. Appareil de radiodiagnostic suivant la revendication 3, caractérisé par le fait que le barre (15) est réalisée sous la forme d'une crémaillère qui est déplacée au moyen d'un pignon (16) entraîné à la main ou par un moteur électrique.

5. Appareil de radiodiagnostic suivant la revendication 1, caractérisé par le fait que les marques prévues pour les positions réglables (I, II, III) comportent des organes d'affichage optique, de préférence des lampes-témoins (35 à 37).

6. Appareil de radiodiagnostic suivant la revendication 5, caractérisé par le fait que, également les sélecteurs de position (29 à 31) contiennent des organes d'affichage optique, de préférence des lampes-témoins (32 à 34) qui répondent lors de l'actionnement des sélecteurs de position (29 à 31).

7. Appareil de radiodiagnostic suivant l'une des revendications 1 à 6, caractérisé par le fait que le panneau d'ensemble (14) est disposé sur la face supérieure d'un boîtier (13) réalisé sous la forme d'un pupitre.

8. Appareil de radiodiagnostic suivant la revendication 7, caractérisé par le fait que le boîtier (13) comporte une partie horizontale (27), dans laquelle les marques des différentes positions (I, II, III) sont disposées, tandis que les sélecteurs de position (29 à 31) sont disposés dans la partie inclinée (28).

9. Appareil de radiodiagnostic suivant l'une des revendications 1 à 8, caractérisé par le fait que dans le panneau d'ensemble (14) se trouve disposées des flèches lumineuses (38, 39), qui indiquent la direction de déplacement de l'appui-tête (7) pendant le réglage.

## Claims

1. Dental X-ray diagnostic apparatus having a pivotably mounted supporting device for an X-ray tube (4) and a film carrier (6) and having arranged therebetween a head support (7) which serves for the support or resting of the head (8) of a patient and which, in order that the head can be fixed in different positions for photographing different objects, is adjustably arranged in the horizontal plane opposite a support (10), characterized in that the adjustable positions (I, II, III) of the head support (7) corresponding to the different objects to be photographed, are marked on a viewing panel (14), that each position can be selected by means of a position selector (29 to 31) which is arranged in the viewing panel (14), and that the position selectors (29, 30, 31) are connected to a scanning device (19 to 25) which is provided with signal transmitters (23, 24, 25) and by means of which the position of the head support (7) with respect to its support (26) is determined, and which when a selected position (I to III) is reached, transmits a signal to an indicating element (35 to 37) which is arranged in the viewing panel (14) and which is assigned to that particular position selector (29 to 31).

2. X-ray diagnostic apparatus as claimed in Claim 1, characterised in that there is provided a mechanical scanning device (19 to 25) having electric switches (23 to 25) as signal transmitters.

3. X-ray diagnostic apparatus as claimed in Claim 2, characterised in that the scanning device (19 to 25) essentially comprises a switch cam (19) arranged on a bar (15) which supports the head support (7), and switch levers (20 to 22) which are arranged on the holder (26) and which cooperate with microswitches (23 to 25).

4. X-ray diagnostic apparatus as claimed in Claim 3, characterised in that the bar (15) is formed as a toothed rack which can be adjusted by means of a toothed wheel (16) operated by hand or by means of an electric motor.

5. X-ray diagnostic apparatus as claimed in Claim 1, characterised in that the markings for the adjustable positions (I, II, III) display optical indicating elements, preferably indicating lamps (35 to 37).

6. X-ray diagnostic apparatus as claimed in Claim 5, characterised in that the position selectors (29 to 31) include optical indicating elements, preferably indicating lamps (32 to 34) which respond on operation of the position selectors (29 to 31).

7. X-ray diagnostic apparatus as claimed in one of Claims 1 to 6, characterised in that the viewing panel (14) is arranged on the upper side of desk-shaped housing (13).

8. X-ray diagnostic apparatus as claimed in Claim 7, characterised in that the housing (13) has a horizontal part (27) in which the markings of the individual positions (I, II, III) are arranged, and that the position selectors (29 to 31) are arranged in the sloping part (28).

9. X-ray diagnostic apparatus as claimed in one of Claims 1 to 8, characterised in that in the viewing panel (14), there are arranged illuminated arrows (38, 39) which display the direction of movement of the head support (7) during adjustment.

FIG 1

FIG 3

FIG 2

FIG 4